# DEMANDE DE BREVET EUROPEEN

(11) **EP 3 854 377 A1**
(43) Date de publication de la demande: **28.07.2021**
(21) Numéro de dépôt: 20305051.3
(22) Date de dépôt: 22.01.2020
(51) Int. Cl.: A61K 8/73, A61K 8/34, A61Q 19/08, A61Q 19/00

(54) **COMPOSITION COMPRENANT DE L'ACIDE HYALURONIQUE ET UN POLYOL OU DE CARBOXYMÉTHYLCELLULOSE**

(71) Demandeur: LABORATOIRES GENEVRIER SAS, 06600 Antibes (FR)
(72) Inventeur: GUILLEMIN, Yannis, 83440 Montauroux (FR); VACHER, Dominique, 6942 Savosa (CH); GOUZE, Jean-Noël, 06220 Vallauris (FR)
(74) Mandataire: Santarelli

(57) **Abrégé**

L'invention a pour objet une composition comprenant au moins de l'acide hyaluronique et au moins un polyol et/ou de la carboxyméthylcellulose (CMC). L'invention a également pour objet l'utilisation d'au moins un polyol et/ou de carboxyméthylcellulose pour stabiliser l'acide hyaluronique dans une composition.

## Description

L'acide hyaluronique (AH) est un glycosaminoglycane (GAG) de haut poids moléculaire constitué d'une répétition de disaccharide de N-acétylglucosamine et d'acide glucuronique.

Son poids moléculaire important peut varier de 10⁵ à 10⁷ Daltons et le polymère peut s'étendre sur une longueur de 2 à 25 µm.

Chez le rat, la moitié de l'acide hyaluronique est retrouvée dans la peau, et un quart provient du squelette et des articulations. Le reste de l'acide hyaluronique est retrouvé dans les muscles et les viscères (Tableau 1, d'après Reed et al. (Reed, 1988)).

**Tableau 1**

| | **Poids (g)** | **acide hyaluronique total (mg)** | **acide hyaluronique (%)** |
|---|---|---|---|
| Rat entier | 200 | 60.5 | 100 |
| Peau | 40.2 | 33.8 | 56 |
| Muscles | 35.7 | 4.69 | 8 |
| Squelette et tissus de soutient | 57.6 | 16.2 | 27 |
| Intestins et estomac | 15.8 | 0.5 | 1 |
| Organes internes restants | 43.4 | 5.25 | 9 |

Chez l'homme en particulier, l'acide hyaluronique est principalement présent au niveau de la matrice extracellulaire. Ses fonctions biologiques incluent la viscoélasticité de tissu conjonctif liquide comme l'articulation synoviale et le fluide vitreux de l'œil, le contrôle de l'hydratation du tissu et le transport de l'eau, l'assemblage supramoléculaire de protéoglycanes de la matrice extracellulaire, et les rôles médiés par des récepteurs sur le détachement cellulaire, la mitose, la migration, le développement tumoral et les métastases et l'inflammation.

Des études sur la cicatrisation montrent que l'acide hyaluronique intervient dans la régulation de l'inflammation, augmente la prolifération des fibroblastes et de kératinocytes et la synthèse de collagène.

Dans la peau adulte, l'acide hyaluronique est dégradé en fragments de différentes tailles par l'activité enzymatique de hyaluronidases et les espèces réactives de l'oxygènes et de l'azote (ROS/RNS). Ces fragments peuvent stimuler les principaux aspects de la réparation cicatricielle comme la contraction de la plaie, l'inflammation, la néoangiogénèse, la fibroplasie, la différenciation de myofibroblaste et l'augmentation de la production/réticulation de collagène.

Les propriétés biologiques de l'acide hyaluronique dépendent de sa taille. En effet, les petits fragments d'acide hyaluronique vont stimuler l'angiogénèse alors que l'acide hyaluronique de haut poids moléculaire l'inhibe. L'acide hyaluronique de haut poids moléculaire va favoriser la différentiation des monocytes en fibrocytes alors que l'acide hyaluronique de bas poids moléculaire va l'inhiber. L'acide hyaluronique de bas poids moléculaire améliore l'autodéfense de la peau contre les microorganismes en induisant la libération de β-defensine 2 par les kératinocytes. Enfin, l'acide hyaluronique de poids moléculaire intermédiaire (250 kDa) favorise la cicatrisation de souris âgées. Cette amélioration de la cicatrisation avec l'acide hyaluronique de 250 kDa passe par une augmentation de l'expression de l'ARNm des récepteurs de l'acide hyaluronique (CD44 et RHAMM), ainsi que du collagène de type I et de type III.

Alors que les autres glycosaminoglycannes sont attachés de façon covalente à une chaîne protéique dans l'appareil de Golgi, l'acide hyaluronique est synthétisé de façon tout à fait unique à la face interne de la membrane cellulaire, le polymère naissant étant extrudé au travers de la membrane vers l'extérieur au fur et à mesure qu'il s'allonge par addition alternée d'un acide glucuronique et d'une unité N-acétylglucosamine. Ce mode de synthèse permet donc une croissance non limitée du polymère qui ne pourrait se dérouler dans le Golgi ou le réticulum endoplasmique sans détruire la cellule en raison de sa taille. Une famille multigénique d'enzymes, les hyaluronansynthases (HAS) sont responsable de sa synthèse. Les trois HAS se distinguent par leur expression temporelle au cours du développement, par leur activité spécifique et par la taille des polymères d'acide hyaluronique qu'elles génèrent. L'acide hyaluronique est synthétisé par les cellules mésenchymateuses, épithéliales et immunitaires ainsi que par les cellules souches mésenchymateuses et hématopoïétiques.

Le renouvellement rapide de l'acide hyaluronique est dû, en partie, à son drainage à partir des tissus d'où il est produit vers les vaisseaux lymphatiques où environ 85% est dégradé. Dans les tissus à structure dense comme le squelette et le cartilage, il est probable que la plupart du renouvellement de l'acide hyaluronique se produit par dégradation métabolique *in situ.* Dans la peau et les articulations, 20-30% du renouvellement de l'acide hyaluronique se produit par métabolisme local et le reste est éliminé par les voies lymphatiques. La demi-vie de l'acide hyaluronique est comprise entre une demi-journée à 2-3 jours indépendamment de ses voies d'élimination. Dans la circulation sanguine, environ 85-90% est éliminé par le foie et 10% par les reins qui n'excrètent que 1-2% dans les urines.

L'acide hyaluronique joue tout d'abord un rôle important dans l'homéostasie tissulaire et l'intégrité biomécanique par ses caractéristiques hydrodynamiques remarquables, particulièrement sa viscosité et sa capacité à retenir l'eau. L'acide hyaluronique permet également l'interaction avec des protéoglycanes et d'autres macromolécules de la matrice extracellulaire et péricellulaire. Il interagit avec la surface cellulaire soit directement via des récepteurs spécifiques (dont CD44, RHAMM (Receptor for Hyaluronic-Acid-Mediated Mobility) et LYVE-1 (Lymphatic Vascular Endothelial hyaluronan receptor)) ou indirectement par l'interaction de ces récepteurs à d'autres récepteurs membranaires.

La transduction du signal induit par la stimulation de CD44 par l'acide hyaluronique joue un rôle :
- Dans la prolifération et la migration cellulaire, d'où son intérêt dans la cicatrisation cutanée, mais également dans la prolifération tumorale
- Dans l'angiogénèse, qui sera en fonction de la taille de l'acide hyaluronique stimulée (acide hyaluronique de bas poids moléculaire ou oligo-acide hyaluronique) ou inhibée (acide hyaluronique de haut poids moléculaire).
- Dans la régulation de l'inflammation (recrutement de cellules inflammatoires et sécrétion de cytokines) via son interaction avec les récepteurs Toll-Like (TLR) 4, TLR2 et CD44

Les injections d'acide hyaluronique dans le derme ont été montrées comme stimulant la synthèse *de novo* de composants de la matrice extracellulaire. Par exemple, sur une peau atrophique, le traitement par l'acide hyaluronique augmente l'expression du collagène et de l'élastine. De même, l'injection d'acide hyaluronique dans le derme de patients âgés stimule la synthèse de collagène de type I, mais pas chez de jeunes patients dont la peau est non soumise au photo-vieillissement.

La peau est le réservoir le plus important d'acide hyaluronique de l'organisme (Tableau 1).

La quantité d'acide hyaluronique dans le derme est très largement supérieure à celle contenue dans l'épiderme et représente environ 50% de l'acide hyaluronique total de l'organisme (Tableau 1). Le derme papillaire est plus riche que le compartiment réticulaire indiquant que le fibroblaste du derme papillaire a une capacité de synthèse de l'acide hyaluronique élevée, similaire à celle des fibroblastes synoviaux.

Il est cependant intéressant de noter que pratiquement la totalité de l'acide hyaluronique a disparu de l'épiderme dans la peau sénile alors qu'il persiste dans le derme âgé suggérant que la régulation de son homéostasie dépend de mécanismes différents dans le derme et l'épiderme. Cependant, si le niveau total d'acide hyaluronique dans le derme reste relativement constant avec l'âge, sa qualité se modifie. La taille du polymère se réduit et il devient moins extractible, suggérant une plus forte association avec les structures tissulaires et peut-être avec un autre répertoire d'hyaladhérines. Ces altérations qualitatives pourraient être responsables de la perte d'hydratation observée dans la peau sénescente. Par ailleurs, si une exposition courte aux UV induit transitoirement un dépôt accru d'acide hyaluronique et une légère réaction oedémateuse, l'exposition répétée aux UV déclenche une réponse de type cicatriciel. Les GAGs trouvés dans la peau photo-vieillie sont similaires à ceux présents dans le tissu cicatriciel, avec une proportion réduite d'acide hyaluronique en faveur de protéoglycannes riches en chondroïtine sulfate. Les radicaux libres générés par les UV-B pourraient détruire les polymères d'acide hyaluronique et générer des fragments biologiquement actifs, pro-inflammatoires et pro-angiogènes. Le niveau de synthèse de l'acide hyaluronique est maintenant facilement monitoré en mesurant l'expression des gènes des HAS présentes dans la peau. Leur expression est stimulée par le TGFβ aussi bien dans le derme que dans l'épiderme, mais avec des cinétiques différentes. D'autres facteurs de croissance, tel le PDGF, ont également une activité stimulante. Par contre, l'expression des HAS et donc la production d'acide hyaluronique, est pratiquement complètement éteinte par les glucocorticoïdes.

On comprend de ce qui précède que l'activité de l'acide hyaluronique est fonction de la taille de ses fragments une fois dégradé, l'acide hyaluronique de haut poids moléculaire ayant une meilleure activité que de l'acide hyaluronique de bas poids moléculaire.

Il est connu d'utiliser l'acide hyaluronique dans des compositions cosmétiques ou dermatologiques destinées à une application topique.

Cependant, l'acide hyaluronique présente une certaine instabilité. Aussi, lorsqu'il est introduit dans une composition cosmétique, son efficacité diminue au cours du temps du fait de sa dégradation. En outre, la composition dans laquelle il est introduit, après un certain temps de stockage, présente des signes de dégradation : coloration, odeur, qui sont inacceptables pour l'utilisateur.

Il pourrait donc être intéressant de disposer d'une composition, particulièrement une composition cosmétique et/ou pharmaceutique, dans laquelle l'acide hyaluronique pourrait être protégé, au moins partiellement, de la dégradation, afin que l'acide hyaluronique contenu dans ladite composition présente une bonne activité.

C'est un des buts de la présente invention.

En effet la demanderesse a maintenant découvert de manière surprenante, après de longs et laborieux travaux que les polyols et/ou la carboxyméthylcellulose (CMC) pouvaient avoir un effet protecteur de l'acide hyaluronique permettant ainsi d'éviter sa dégradation et lui conservant dans le temps une stabilité accrue gage d'une activité meilleure sur une longue période en évitant, à tout le moins en ralentissant, sa dégradation.

Ainsi l'invention a pour objet premier une composition, avantageusement une composition cosmétique ou pharmaceutique, comprenant au moins de l'acide hyaluronique et au moins un polyol et/ou de la carboxyméthylcellulose (CMC). Préférentiellement la composition selon l'invention peut comprendre, en plus de l'acide hyaluronique, au moins un polyol et de la carboxyméthylcellulose.

Selon l'invention, l'acide hyaluronique pourra être un acide hyaluronique de poids moléculaire compris entre 10⁵ à 10⁷, préférentiellement entre 10⁵ et 4.10⁶ très préférentiellement entre 5.10⁵ 2.10⁶Da.

Par polyol, on désigne un composé de type alkyle, linéaire ramifié ou cyclique, saturé ou insaturé portant au moins deux fonctions -OH sur la chaine alkyle, ainsi que les polymères (polyéthers) de ces composés alkyles polyhydroxylés. De préférence il s'agit d'un composé alkyle ayant de 2 à 12 atomes de carbone, et encore plus préférentiellement de 2 à 8 atomes de carbone. Avantageusement, ce composé alkyle comporte 2 ou 3 atomes de carbone.

Selon l'invention, l'acide hyaluronique pourra être présent dans la composition en une quantité comprise entre 0,01% et 20% du poids total de la composition, préférentiellement entre 0,03% et 10% du poids total de la composition, très préférentiellement entre 0,05% et 1% du poids total de la composition.

Selon l'invention le polyol peut être choisi parmi l'éthylène glycol [(HOCH₂-CH₂OH)], le diéthylène glycol [(HOCH₂-CH₂-O-CH₂-CH₂OH)], le triéthylène glycol [(HOCH₂-CH₂-O-CH₂-CH₂OCH₂-CH₂OH], le propylène glycol [(propane-1,2-diol : HOCH₂-CHOH-CH₃)], le triméthylène glycol [(propane-1,3-diol : HOCH₂-CH₂-CH₂OH)], le propylène glycol, les polymères et les copolymères du glycérol, de l'éthylène glycol et du propylène glycol, comme par exemple le dipropylène glycol et l'hexaglycérol ; l'hexylène glycol, le pentylène glycol, le butyldiglycol, le 1,2,3trihydroxyhexane, le butylène glycol [(butane-1,3-diol], le n-butylène glycol [(butane-1,4-diol], le 2,3-butylène glycol [ou secbutylène glycol (butane-2,3 diol)], encore parmi les Triols, par exemple le Glycérol ; les Tétraols comme par exemple l'érythritol, le thréitol ; les pentols (pentanols) comme par exemple le xylitol, l'arabitol (lyxitol), le ribitol (adonitol) ; les hexols comme par exemple le sorbitol (gulitol), le dulcitol (galactitol), le mannitol, le fucitol, l'iditol ; les heptols comme par exemple le volemitol ; ou encore l'isomalt, le maltitol, l'isomaltitol, le lactitol (lactositol), le maltotriitol, le maltotétraitol, le polyglycitol. Préférentiellement selon l'invention, le polyol peut être choisi parmi les triols et les hexols, très préférentiellement parmi le glycérol, le sorbitol et le mannitol. Selon l'invention encore, le polyol pourra être présent dans la composition en une quantité comprise entre 0,05% et 90% du poids total de la composition, préférentiellement entre 0,5% et 80% du poids total de la composition, très préférentiellement entre 1,0 et 75% du poids total de la composition.

Selon l'invention, la carboxyméthylcellulose pourra être présente dans la composition en une quantité comprise entre 0,1% et 72% du poids total de la composition, préférentiellement entre 0,5% et 50% du poids total de la composition, très préférentiellement entre 1 et 5% du poids total de la composition.

Toujours selon l'invention le rapport entre l'acide hyaluronique et le polyol pourra être dans la composition compris entre 0,0001 et 400 préférentiellement entre 0,0003 et 2 très préférentiellement entre 0,0006 et 1

De même selon l'invention le rapport entre l'acide hyaluronique et la carboxyméthylcellulose pourra être dans la composition compris entre 0,0001 et 200 préférentiellement entre 0,0006 et 20 et très préférentiellement entre 0,01 et 1.

Toujours selon l'invention le rapport entre le polyol et la carboxyméthylcellulose pourra être dans la composition compris entre 0,0007 et 900, préférentiellement entre 0,0001 et 160, très préférentiellement entre 0,2 et 75.

Selon un deuxième objet, l'invention a pour objet l'utilisation d'au moins un polyol et/ou de carboxyméthylcellulose pour ralentir, limiter, voire annihiler la dégradation de l'acide hyaluronique, avantageusement dans une composition, particulièrement une composition cosmétique ou pharmaceutique.

Selon un troisième objet, l'invention a pour objet l'utilisation d'au moins un polyol et/ou de carboxyméthylcellulose pour ralentir, limiter, voire annihiler, avantageusement dans une composition, particulièrement une composition cosmétique ou pharmaceutique la dégradation de l'acide hyaluronique induite par les radiations ionisantes comme par exemple les radiations de types béta, gamma ou les rayonnements Ultra-Violets.

Selon un quatrième objet l'invention a pour objet l'utilisation d'au moins un polyol et/ou de carboxyméthylcellulose pour ralentir, limiter, voire annihiler la dégradation de l'acide hyaluronique induite par le stress oxydatif, avantageusement dans une composition, particulièrement une composition cosmétique ou pharmaceutique.

Selon un cinquième objet l'invention a pour objet l'utilisation d'au moins un polyol et/ou de carboxyméthylcellulose pour ralentir, limiter, voire annihiler la dégradation de l'acide hyaluronique présent dans une composition destinée à favoriser la cicatrisation.

Selon un sixième objet l'invention a pour objet l'utilisation d'au moins un polyol et/ou de carboxyméthylcellulose pour ralentir, limiter, voire annihiler la dégradation de l'acide hyaluronique présent dans une composition destinée à moduler l'inflammation.

Selon un septième objet l'invention a pour objet l'utilisation d'au moins un polyol et/ou de carboxyméthylcellulose pour ralentir, limiter, voire annihiler la dégradation de l'acide hyaluronique présent dans une composition destinée à augmenter la prolifération des fibroblastes et/ou des kératinocytes

Selon un huitième objet l'invention a pour objet l'utilisation d'au moins un polyol et/ou de carboxyméthylcellulose pour ralentir, limiter, voire annihiler la dégradation de l'acide hyaluronique présent dans une composition destinée à stimuler la synthèse de collagène.

Selon un neuvième objet l'invention a pour objet l'utilisation d'au moins un polyol et/ou de carboxyméthylcellulose pour ralentir, limiter, voire annihiler la dégradation de l'acide hyaluronique présent dans une composition destinée à inhiber l'angiogenèse.

Selon un dixième objet l'invention a pour objet l'utilisation d'au moins un polyol et/ou de carboxyméthylcellulose pour ralentir, limiter, voire annihiler la dégradation de l'acide hyaluronique présent dans une composition destinée à favoriser la différentiation des monocytes en fibrocytes.

Selon un onzième objet l'invention a pour objet l'utilisation d'au moins un polyol et/ou de carboxyméthylcellulose pour ralentir, limiter, voire annihiler la dégradation de l'acide hyaluronique présent dans une composition destinée à traiter le vieillissement cutané.

Selon un douzième objet l'invention a pour objet l'utilisation d'au moins un polyol et/ou de carboxyméthylcellulose pour ralentir, limiter, voire annihiler la dégradation de l'acide hyaluronique présent dans une composition destinée à traiter les rides, particulièrement au comblement des rides.

Selon un treizième objet l'invention a pour objet l'utilisation d'au moins un polyol et/ou de carboxyméthylcellulose pour ralentir, limiter, voire annihiler la dégradation de l'acide hyaluronique présent dans une composition destinée à être utilisée en mésothérapie, particulièrement dans la réhydratation/réjuvénation des tissus.

On comprend donc que selon l'invention la composition peut comprendre, outre l'acide hyaluronique, au moins un polyol seul ou au moins de la carboxyméthylcellulose seule ou un mélange d'au moins un polyol et de carboxyméthylcellulose. Préférentiellement selon l'invention la composition peut comprendre un mélange d'au moins un polyol et de carboxyméthylcellulose.

La composition de l'invention peut se présenter sous toutes les formes galéniques connues, avantageusement pour une application topique, notamment sous forme d'une solution aqueuse, hydroalcoolique ou huileuse, d'une émulsion huile dans-eau ou eau-dans-huile ou d'une émulsion multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooings ou après-shampooings.

De façon connue et sans limitation, la composition de l'invention peut contenir les adjuvants habituels dans les domaines cosmétique et dermatologique, tels que les gélifiants hydrophiles ou lipophiles, les actifs hydrophiles ou lipophiles, les conservateurs, les antioxydants, les solvants organiques ou inorganiques, les parfums, les charges, les filtres, les pigments, les absorbeurs d'odeur et les matières colorantes, les corps gras, les épaississants ioniques ou non ioniques, les adoucissants, les agents anti-radicaux libres, les opacifiants, les stabilisants, les émollients, les silicones, les α-hydroxyacides, les agents anti-mousse, les agents hydratants, les vitamines, les tensioactifs, les séquestrants, les polymères, les propulseurs; les agents alcalinisants ou acidifiants, ou tout autre ingrédient habituellement utilisé dans le domaine.

Les quantités de ces différents adjuvants pourront être celles classiquement utilisées dans les domaines considérés et par exemple de 0,001 à 90%, préférentiellement de 1,0% à 75%, du poids total de la composition. Ces adjuvants, selon leur nature, peuvent être introduits dans la phase grasse, dans la phase aqueuse, dans les vésicules lipidiques et/ou dans les nanoparticules. Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Comme huiles, on peut citer les huiles minérales (vaseline); végétales (huile d'amande douce, de macadamia, de pépin de cassis, de jojoba) ; synthétiques comme le perhydrosqualène, les alcools, tes acides ou les esters gras (comme le benzoate d'alcools en C12-C15 vendu sous la dénomination commerciale "Finsolv TN" par la société Finetex, le palmitate d'octyle, le lanolate d'isopropyle, les triglycérides dont ceux des acides caprique/caprylique), les esters et éthers gras oxyéthylénés ou oxypropylénés; silicosées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools.

Les épaississants peuvent être choisis notamment parmi les acides polyacryliques réticulés, les gommes de guar et celluloses modifiées ou non telles que la gomme de guar, hydroxypropylée, la méthylhydroxyéthylcellulose et l'hydroxypropylméthyl cellulose, agents de coloration de la peau comme par exemple les dérivés mono ou polycarbonylés tels que l'isatine, l'alloxane, la ninhydrine, le glycéraldéhyde, l'aldéhyde mésotartrique,

Bien entendu, l'homme de l'art veillera à choisir le ou les éventuels composés complémentaires cités ci-dessus et/ou leurs quantités de manière telle que les propriétés avantageuses attachées intrinsèquement à l'utilisation de l'acide hyaluronique conforme à l'invention ne soient pas, ou substantiellement pas, altérées par la ou les adjonctions envisagées. Les compositions selon l'invention peuvent être préparées selon les techniques bien connues de l'homme de l'art, en particulier celles destinées à la préparation d'émulsions de type huile-dans-eau (H/E) ou eau-dans-huile (E/H). Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray. De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

D'autres caractéristiques de l'invention pourront apparaitre dans les exemples concrets, mais nullement limitatifs, illustrant l'invention, exposés ci-après et dans lesquels

La figure 1 présente les résultats obtenus lors de l'étude par chromatographie par perméation de gel (GPC : Gel Permeation Chromatography) de la dégradation d'acide hyaluronique dans des compositions comprenant un polyol et de la carboxyméthylcellulose (CMC), soumises, ou non, à un stress de type radiation ionisante.

La figure 1a présente les résultats de l'analyse par GPC de la dégradation de l'acide hyaluronique dans des compositions non-irradiées.

La figure 1b présente les masses molaires (MW) des AH en fonction de leur association avec les polyols type mannitol, sorbitol, glycérol et CMC.

La figure 1c présente les résultats de l'analyse de la dégradation de l'acide hyaluronique en condition de stress induit par une dose d'irradiation eBeam de 8 à 12 kGy et préalablement associés ou pas à différents polyols ou à la CMC.

La figure 1d présente l'évolution des masses molaires (MW) de l'AH, en condition de stress induit par une dose d'irradiation eBeam de 8 à 12 kGy et en fonction de leur association avec les polyols type mannitol, sorbitol, glycérol et CMC. La flèche indique un déplacement vers la masse moléculaire plus faible (temps de rétention plus élevé).

La figure 1e présente les résultats de l'analyse de la dégradation de l'acide hyaluronique en condition d'irradiation eBeam à 12-25 kGy.

La figure 1f présente l'évolution les masses molaires (MW) des AH, en condition de stress type irradiation eBeam à une dose de 12 à 25 kGy et en fonction de leur association avec les polyols type mannitol, sorbitol, glycérol et CMC. La flèche indique un déplacement vers la masse moléculaire plus faible (temps de rétention plus élevé).

La figure 1g présente les résultats de l'analyse de la dégradation de l'acide hyaluronique en condition d'irradiation eBeam à 25-50 kGy.

La figure 1h présente l'évolution les masses molaires (MW) des AH, en condition de stress type irradiation eBeam à une dose de 25 à 50 kGy et en fonction de leur association avec les polyols type mannitol, sorbitol, glycérol et CMC. La flèche indique un déplacement vers la masse moléculaire plus faible (temps de rétention plus élevé).

La figures 2 présente les profils d'élution des standards d'acide hyaluronique références dans le contexte de l'effet du stress oxydatif.

La figures 3 présente les effets du stress oxydatif sur la masse moléculaire de l'acide hyaluronique en présence ou non de polyols en fonction du temps.

### Exemple 1 : Evaluation comparative de l'effet d'excipients sur l'acide hyaluronique avant et après irradiation

Le but de cette expérimentation est d'évaluer comparativement la masse moléculaire de l'acide hyaluronique mis en solution dans différentes solutions d'excipients, avant et après irradiation à différentes doses.

### 1. Principe

L'acide hyaluronique est mis en solution dans différentes solutions d'excipients afin d'étudier leur effet sur sa masse moléculaire avant et après irradiation Ebeam à trois doses différentes (8-12 kGy, 12-25 kGy et 25-50 kGy).

### 2. Matériels et Méthodes

### a. Matériels

∘ Tubes verre 5mL + bouchons + capsules métal
∘ Vials 1mL GPC (Agilent réf 5188-6593)
∘ Filtre GPC acide hyaluronique (PTFE 13mm 0.2µm Agilent 51905265)
∘ Balance Sartorius (N°4)

### b. Réactifs

∘ Acide hyaluronique (Altergon lot 9998 - 3M Da)
∘ Mannitol (Merck Lot FN 1378803750)
∘ Sorbitol (Roquette Lot E090B)
∘ Glycérol (Sigma Lot SHBK3676)
∘ CMC (Coluxia Lot C161437)
∘ Eau distillée
∘ Eau ultra pure GPC grade
∘ NaCl (Fisher ref 10274392)
∘ Méthanol (Fisher 1851587)
∘ Eau ultra pure GPC grade (Merk ref 1.115333.1000)

### c. Préparation des solutions

L'ensemble des solutions est préparé selon le tableau suivant :

| **acide hyaluronique (mg)** | **Glycérol (g)** | **Mannitol (mg)** | **Sorbitol (mg)** | **CMC (mg)** | **Volume Eau distillée (mL)** |
|---|---|---|---|---|---|
| 200 | - | 1050 | - | - | Qsp 20 |
| 200 | - | - | 1050,00 | - | |
| 200 | 100 | - | - | - | |
| 200 | 0,2 | - | - | - | |
| 200 | - | - | - | 1000 | |
| 200 | - | - | - | - | |

Les solutions ainsi préparées sont ensuite lyophilisées (Cryotec Pilot Compact) selon le protocole comprenant une première étape de congélation par passage d'une température de +25°C à une température de -45°C en 60 minutes, puis maintien à -45°C pendant 6 heures ; une deuxième étape de sublimation par passage d'une température de -45°C à une température de -20°C (sous vide 0,16 mBar), pendant 4h00, puis maintien à -20°C pendant 24h00 (sous vide 0,16mBar) ; une troisième étape de séchage secondaire par passage d'une température de 20°C à 25°C en 4 heures (sous vide 0,007 mBar) et maintien à la température de +25°C pendant 15 heures (sous vide 0,007mBar)

A l'issue de la lyophilisation les échantillons sont alors soumis à irradiation de type Béta dans un appareil Mevex A29, 34 kW, 10 Mev à une fréquence de 640 Hz, avec une consigne de balayage de 2,7A, pendant un nombre de tours, à une vitesse de 2,51 m/min, aux doses suivantes : 0, 8-12, 17.5-25 et 25-50 kGy.

### 3. Analyses GPC

### 3.a Préparation des échantillons pour analyse de l'acide hyaluronique

Avant et après irradiation, chaque solution préparée est analysée à l'GPC selon le protocole suivant :
Une dilution dans un mélange de NaCl 2M/MeOH 2% et réalisée si nécessaire afin d'obtenir une concentration finale d'acide hyaluronique comprise entre 0,5 et 1g/L.

Les échantillons sont alors filtrés et dispensés dans des fioles pour l'analyse à l'GPC.

### 3.b Analyse de l'acide hyaluronique :

L'analyse de la masse moléculaire de l'acide hyaluronique (entre 64 kDa et 1,3 MDa) et réalisé dans un appareil SL1200 de marque Agilent selon le protocole suivant :
Colonnes : TSKgel GMPWXL et colonne de garde associée ;
Température des colonnes/détecteur : 45°C ;
Débit : 0,5 mL/min NaCl 0,2 M et MeOH 2% ;
Détecteurs :
   Index de réfraction (RID, Refractive Index Detector) → Température : 45°C
   Détecteurs à barrette de diodes. (DAD, Diod Array Detector) → Longueur d'onde : 205 nm
   Volume d'injection : 50 µL

### 3.c Résultats analyse GPC

### 3.c.1 Gamme

La gamme de référence suivante est réalisée.

| **Point** | **Temps de rétention à l'apex du pic (mins)** | **Poids moléculaire (MW)** | **Log MW** | **% d'erreur** |
|---|---|---|---|---|
| 1 | 15,80940 | 1300000 | 6,11 | -29,42 |
| 2 | 17,16300 | 720000 | 5,86 | 21,25 |
| 3 | 17,82000 | 390000 | 5,59 | 14,25 |
| 4 | 17,82000 | 1 | 0,00 | -33440718,57 |
| 5 | 18,79920 | 210000 | 5,32 | 27,50 |
| 6 | 19.07820 | 90000 | 4.95 | -35.19 |
| 7 | 19.57320 | 77000 | 4.89 | -6.16 |
| 8 | 19.83960 | 60000 | 4.78 | -9.98 |

Le coefficient de détermination R2 présente la valeur 0.958192 ; la gamme est validée

### 3.c.2 Echantillons

Le tableau ci-dessous présente les résultats obtenus sur les échantillons testés.

| **Echantillons** | **Dose d'irradiatio n** | **Masse molair e à l'Apex Mp** | **Masse molair e en nombr e Mn** | **Masse molair e en masse Mw** | **Polydispersi té PD** |
|---|---|---|---|---|---|
| acide hyaluronique seul | non irradié | 395526 0 | 188960 2 | 395634 5 | 2,1 |
| | 8-12 kGy | 324438 | 27570 | 35356 | 1,28 |
| | 12-25 kGy | 14577 | 8629 | 19307 | 2,2 |
| | 25-50 kGy | 19327 | 8117 | 14915 | 1,84 |
| acide hyaluronique + Mannitol | non irradié | 379278 6 | 204505 6 | 355510 5 | 1,74 |
| | 8-12 kGy | 337323 | 246524 | 340051 | 1,38 |
| | 12-25 kGy | 174929 | 71073 | 165084 | 2,32 |
| | 25-50 kGy | 91903 | 72292 | 100650 | 1,4 |
| acide hyaluronique + Sorbitol | non irradié | 427657 1 | 241177 8 | 399510 9 | 1,66 |
| | 8-12 kGy | 266860 | 218014 | 408302 | 1,87 |
| | 12-25 kGy | 145154 | 78841 | 152422 | 1,93 |
| | 25-50 kGy | 60243 | 40529 | 109265 | 2,7 |
| acide hyaluronique + Glycérol 100 | non irradié | 299185 2 | 208907 3 | 307789 5 | 1,47 |
| | 8-12 kGy | 502827 | 297841 | 598552 | 2,01 |
| | 12-25 kGy | 379800 | 224544 | 394409 | 1,76 |
| | 25-50 kGy | 235647 | 101000 | 215774 | 2,14 |
| acide hyaluronique + Glycérol 0,2 | non irradié | 389846 | 240082 4 | 400330 4 | 1,67 |
| | 8-12 kGy | 278292 | 110160 | 245240 | 2,2 |
| | 12-25 kGy | 71869 | 39110 | 79587 | 2,04 |
| | 25-50 kGy | 44015 | 22988 | 49331 | 2,15 |
| acide hyaluronique + CMC | non irradié | 405372 0 | 185169 5 | 363544 2 | 1,96 |
| | 8-12 kGy | 200695 | 104860 | 404981 | 3,86 |
| | 12-25 kGy | 85739 | 50251 | 300369 | 5,9 |
| | 25-50 kGy | 82814 | 55613 | 172783 | 3,1 |

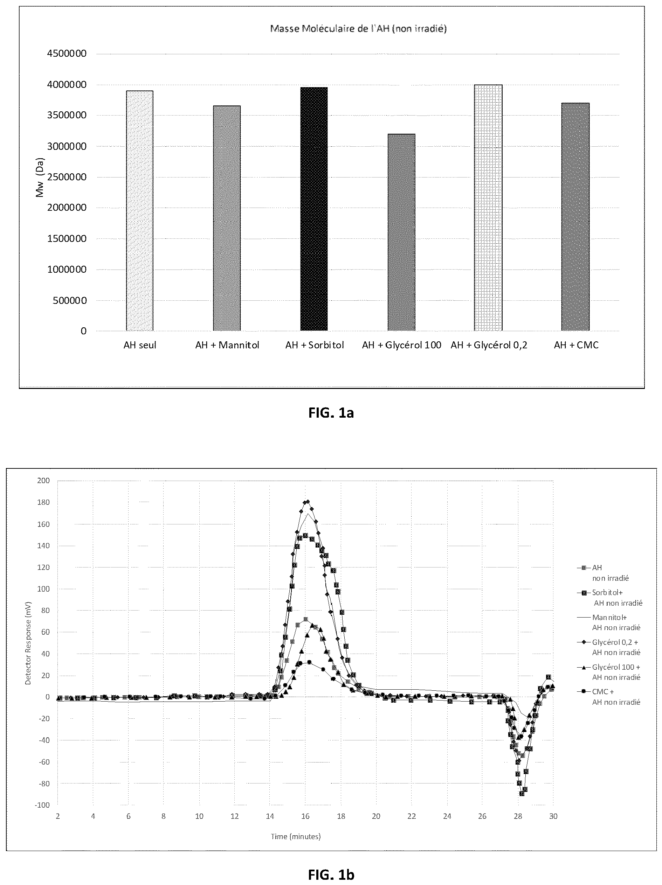

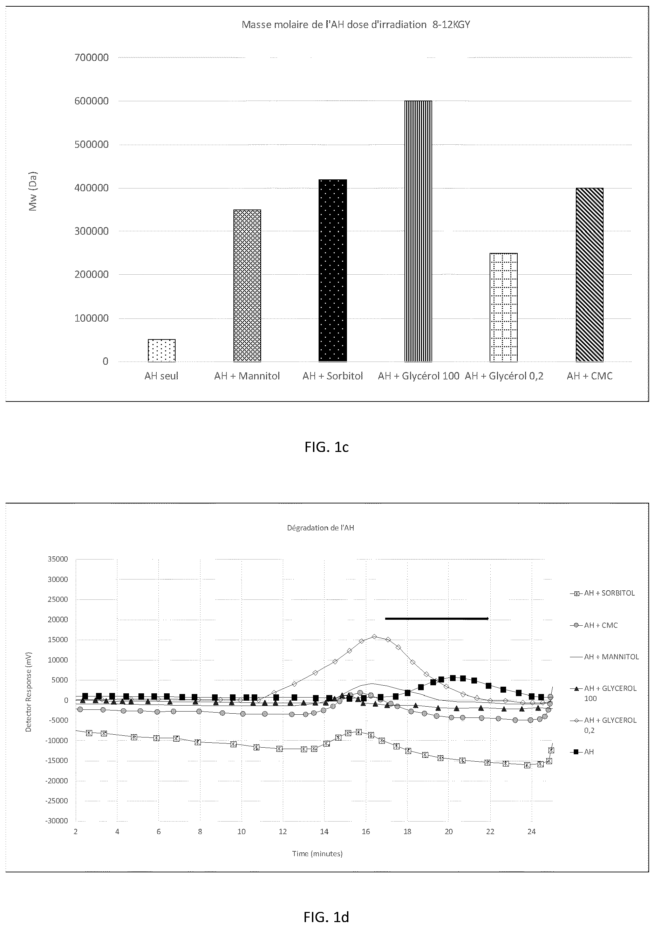

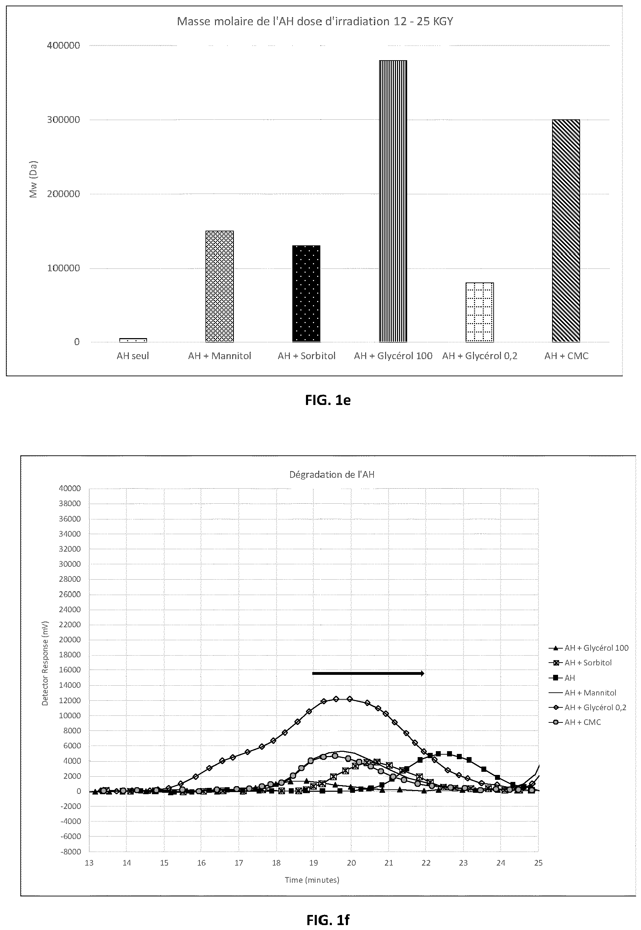

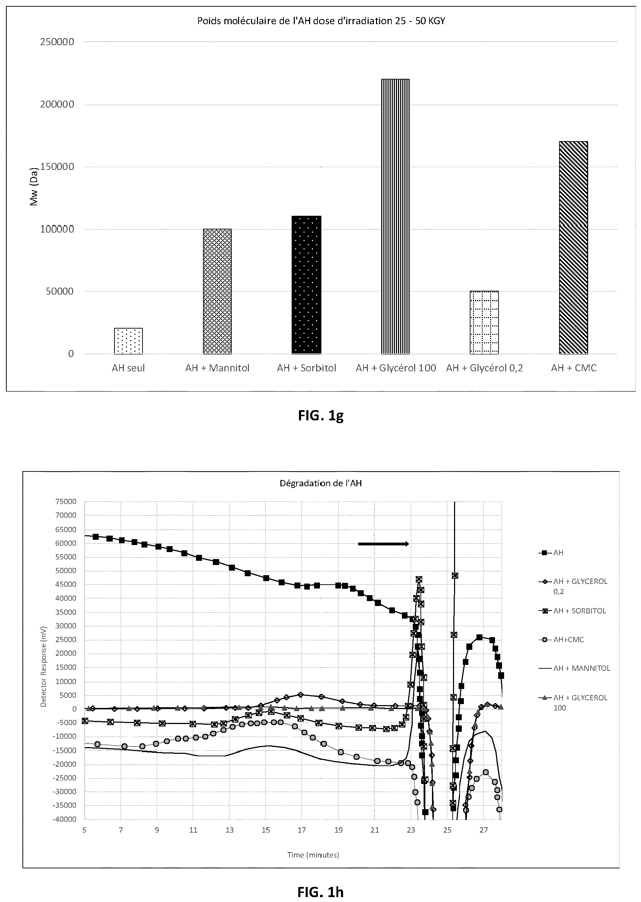

Ces résultats sont également présentés aux figures 1a à 1f

### 4. Conclusions

On observe que :
- Quelle que soit la solution dans laquelle l'acide hyaluronique est remis en suspension, avant irradiation, son poids moléculaire reste stable.
- Plus la dose d'irradiation est importante, plus l'acide hyaluronique est dégradé.
- Un effet protecteur de chaque excipient sur l'acide hyaluronique lors de l'irradiation, quelle que soit la dose, l'effet protecteur le plus important étant observé en présence du glycérol 100 et de CMC.

### Exemple 2 : Evaluation comparative de l'effet des excipients sur l'acide hyaluronique en condition ou non de Stress Oxydatif -

Le but de cette expérimentation est d'évaluer comparativement l'effet du stress oxydatif sur la masse moléculaire de l'acide hyaluronique mis en solution dans différentes solutions d'excipients.

### 1. Principe

L'acide hyaluronique est mis en solution dans différentes solutions d'excipients afin d'étudier leur effet sur sa masse moléculaire en présence ou non de peroxyde d'hydrogène et de chlorure de cuivre d'après le protocole de Chen et al. 2019 (Molecules 2019, 24, 61).

### 2. Matériels et Méthodes

### a. Matériels

∘ Tubes verre 5mL + bouchons + capsules métal
∘ Flacons 1mL GPC (Agilent réf 5188-6593)
∘ Filtre GPC acide hyaluronique (PTFE 13mm 0.2µm Agilent 5190 5265)
∘ Bain-Marie (Memmert 10L)
∘ Balance Sartorius (N°4)

### b. Réactifs

∘ Acide hyaluronique (Altergon lot 9998 - 3M Da)
∘ Mannitol (Merck Lot FN 1378803750)
∘ Sorbitol (Roquette Lot E090B)
∘ Glycérol (Sigma Lot SHBK3676)
∘ CMC (Coluxia Lot C161437)
∘ H₂O₂ (Fisher ref 15632040)
∘ CuCl₂ (Fisher ref 10093650)
∘ Eau distillée
∘ Eau ultra pure GPC grade
∘ NaCl (Fisher ref 10274392)
∘ Méthanol (Fisher 1851587)
∘ De Chlorure de Sodium (NaCl) Eau ultra pure GPC grade (Merk ref 1.115333.1000)

### c Préparation des solutions

### Solution NaCl 0,2M :

La masse moléculaire du NaCl étant de 58,44 g.mol⁻¹, on prépare une solution à 1,17% soit 2,34g dans 200mL d'eau distillée.

Solution H₂O₂ à 3% : réalisé une dilution de la solution mère d'H₂O₂ à 30% d'un facteur 10.

### Solution CuCl₂ à 50mM :

La masse moléculaire du CuCl₂ étant de 170,48 g.mol⁻¹, on pèse 8,5 mg dans 20mL d'eau distillée.

### Préparations des échantillons :

L'ensemble des solutions a été préparé selon le tableau suivant afin d'obtenir une concentration en acide hyaluronique à 0,5% w/v selon le protocole de Chen et al 2019 :

| **Acide hyaluronique (mg)** | **Glycérol (g)** | **Mannitol (mg)** | **Sorbitol (mg)** | **CMC (mg)** | **NaCl 0,2M (mL)** |
|---|---|---|---|---|---|
| 100 | - | 525 | - | - | Qsp 20 |
| 100 | - | - | 525 | - | |
| 100 | 50 | - | - | - | |
| 100 | 0,1 | - | - | - | |
| 100 | - | - | - | 500 | |
| 100 | - | - | - | - | |

### 3. Protocole de stress oxydatif

On prépare pour chaque échantillon et chaque temps 2 tubes de 1mL : 1 sans ajout, 1 auquel sont ajoutés 5 mM de H₂O₂ soit 5,7µL/tube de la solution de H₂O₂ à 3% et 5µM de CuCl₂ soit 2µL/tube de la solution préparée précédemment. Les échantillons en condition de stress oxydatif sont incubés à 50°C pendant 5, 10, 20, 30 et 60 min.

Après chaque temps d'incubation, la réaction est stoppée par congélation rapide à -80°C pour analyse GPC.

### 4. Analyse GPC

### 4.a Préparation des échantillons pour analyse de l'acide hyaluronique

### Préparation de la gamme de standards

Les standards (1 à 7) et les échantillons à analyser sont préparés dans un tampon NaCl 0,2M/MeOH 2% (qui sert d'éluant) à des concentrations comprises entre 0,5 et 1 g/L puis filtrés (0,22 µM) avant d'être échantillonnés en flacons pour analyse GPC.

### Analyses-en GPC (Gel Permeation Chromatography)

L'analyse de la masse moléculaire de l'acide hyaluronique (entre 64 kDa et 1,3 MDa) est réalisée dans un appareil SL 1200 de marque AGILENT selon le protocole suivant :
Colonnes : TSKgel GMPWXL et colonne de garde associée
Température des colonnes/détecteur : 45 °C
Débit : 0,5 mL/min NaCl 0,2 M et MeOH 2%
Détecteurs :
   RID → Température : 45°C
   DAD → Longueur d'onde : 205 nm
   Volume d'injection : 50 µL

### 4.b Résultats analyse GPC

### 4.b.1 Gamme

La gamme de référence suivante est réalisée.

| **Standards** | **Temps de rétention à l'apex du pic (mins)** | **Poids moléculaire (MW)** | **Log MW** | **% d'erreur** |
|---|---|---|---|---|
| 1 | 18,00540 | 1300000 | 6,11 | 12,81 |
| 2 | 18,29880 | 720000 | 5,86 | -22,91 |
| 3 | 19,33920 | 390000 | 5,59 | 5,66 |
| 4 | 20,03220 | 210000 | 5,32 | 2,36 |
| 5 | 20,70720 | 77000 | 4,89 | -50,68 |
| 6 | 21,13920 | 90000 | 4,95 | 10,46 |
| 7 | 21,34260 | 60000 | 4,75 | -13,13 |

Coefficient de détermination : 0.986936 ; Coefficient de corrélation linéaire : - 0.993446 ; Standard Y Erreur E

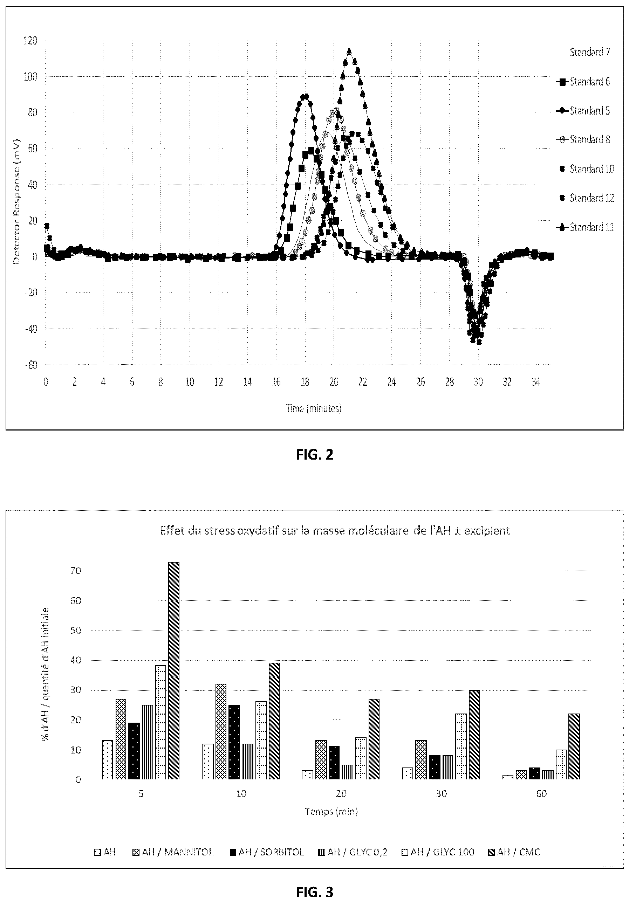

La gamme étalon est conforme avec un R² de 0.99. Les analyses peuvent se poursuivre (voir Figure 2)

### 4.b.2 Echantillon

Le tableau ci-dessous présente les résultats obtenus sur les échantillons testés.

| **Echantillons** | **Temps** | **Mp** | **Mn** | **Mw** | **PD** | **%** |
|---|---|---|---|---|---|---|
| acide hyaluronique seul | T=0 | 1736745 | 958442 | 2055581 | 2,1 | 100,0 |
| acide hyaluronique + 5M H₂O₂/CuCl₂ | T=5min | 220759 | 59767 | 255328 | 4,3 | 12,4 |
| | T=10min | 138791 | 54202 | 232579 | 4,3 | 11,3 |
| | T=20min | 38177 | 11575 | 46620 | 4 | 2,3 |
| | T=30min | 44505 | 18640 | 58951 | 3,2 | 2,9 |
| | T=60Min | 7822 | 2847 | 11561 | 4 | 0,6 |
| acide hyaluronique + mannitol | T=0 | 2187646 | 118176 | 2421190 | 2 | 100,0 |
| acide hyaluronique + mannitol + H₂O₂/CuCl₂ | T=5min | 542153 | 244225 | 656459 | 2,7 | 27,1 |
| | T=10min | 546024 | 249815 | 767406 | 3,072 | 31,7 |
| | T=20min | 117619 | 63292 | 295937 | 4,7 | 12,2 |
| | T=30min | 137950 | 62881 | 275914 | 4,4 | 11,4 |
| | T=60Min | 45738 | 17750 | 88586 | 5 | 3,7 |
| acide hyaluronique + sorbitol | T=0 | 2392690 | 1281982 | 2617233 | 2 | 100,0 |
| acide hyaluronique + sorbitol + H₂O₂/CuCl₂ | T=5min | 415459 | 184625 | 503731 | 2,7 | 19,2 |
| | T=10min | 416701 | 180666 | 565745 | 3,1 | 21,6 |
| | T=20min | 106726 | 52670 | 291150 | 5,5 | 11,1 |
| | T=30min | 97580 | 43554 | 201977 | 4,64 | 7,7 |
| | T=60Min | 34275 | 13963 | 107268 | 7,7 | 4,1 |
| acide hyaluronique + glycérol 0,2 | T=0 | 2049366 | 1046444 | 2214354 | 2,1 | 100,0 |
| acide hyaluronique + glycérol 0,2 + H₂O₂/CuCl₂ | T=5min | 394921 | 185736 | 485579 | 2,6 | 21,9 |
| | T=10min | 197106 | 83178 | 246580 | 2,7 | 11,1 |
| | T=20min | 76416 | 31075 | 102560 | 3,3 | 4,6 |
| | T=30min | 88543 | 35747 | 170932 | 4,9 | 7,7 |
| | T=60Min | 29402 | 10610 | 85505 | 8 | 3,9 |
| acide hyaluronique + glycérol 100 | T=0 | 2530971 | 1516634 | 2743329 | 1,8 | 100,0 |
| acide hyaluronique + glycérol 100 + H₂O₂/CuCl₂ | T=5min | 961714 | 433316 | 1021851 | 2,4 | 37,2 |
| | T=10min | 633176 | 308487 | 704977 | 2,3 | 25,7 |
| | T=20min | 337409 | 162026 | 369177 | 2,3 | 13,5 |
| | T=30min | 481365 | 207910 | 596919 | 2,9 | 21,8 |
| | T=60Min | 164272 | 88322 | 239735 | 2,7 | 8,7 |
| acide hyaluronique + CMC | T=0 | 1335506 | 463913 | 1351801 | 2,9 | 100 |

| | | | | | | |
|---|---|---|---|---|---|---|
| acide hyaluronique + CMC + H₂O₂/CuCl₂ | T=5min | 729745 | 158142 | 987117 | 6,2 | 73,0 |
| | T=10min | 279924 | 73608 | 505860 | 6,9 | 37,4 |
| | T=20min | 272377 | 51313 | 360989 | 7 | 26,7 |
| | T=30min | 260247 | 64023 | 383282 | 6 | 28,4 |
| | T=60Min | 171667 | 38554 | 273637 | 7,1 | 20,2 |

Le tableau suivant présente le pourcentage d'acide hyaluronique dans chaque échantillon après incubation par rapport à la quantité d'acide hyaluronique initiale

| Temps (min) | AH+5M H₂O₂/CuC I₂ | AH /mannitol | AH /sorbitol | AH /glyc 0,2 | AH /glyc 100 | AH /CMC |
|---|---|---|---|---|---|---|
| 0 | 100 | 100 | 100 | 100 | 100 | 100 |
| 5 | 12,4 | 27,1 | 19,2 | 21,9 | 37,2 | 73 |
| 10 | 11,3 | 31,7 | 21,6 | 11,1 | 25,7 | 37,4 |
| 20 | 2,3 | 12,2 | 11,1 | 4,6 | 13,5 | 26,7 |
| 30 | 2,9 | 11,4 | 7,7 | 7,7 | 21,8 | 28,4 |
| 60 | 0,6 | 3,7 | 4,1 | 3,9 | 8,7 | 20,2 |

| | | | | | | |
|---|---|---|---|---|---|---|
| AH: Acide Hyaluronique | | | | | | |

Ces résultats sont également présentés à la figure 3.

### 1. Conclusions

On observe :
- Une dégradation de l'acide hyaluronique seul quasi-totale sur 60min
- À chaque temps, une dégradation plus importante de l'acide hyaluronique seul en comparaison de l'acide hyaluronique en présence d'excipient et ce quel que soit l'excipient étudié.
- Tous les excipients utilisés protègent l'acide hyaluronique de la dégradation liée au stress oxydatif, l'effet protecteur le plus important étant observé en présence du glycérol 100 et de CMC.

## Revendications

1. Composition comprenant au moins de l'acide hyaluronique et au moins un polyol et/ou de la carboxyméthylcellulose (CMC), préférentiellement de l'acide hyaluronique et au moins un polyol et de la carboxyméthylcellulose.

2. Composition selon l'une quelconque des revendications 1 ou 2, **caractérisée en ce que** l'acide hyaluronique a un poids moléculaire compris entre 10⁵ à 10⁷, préférentiellement entre 10⁵ et 4.10⁶ très préférentiellement entre 5.10⁵ 2.10⁶Da.

3. Composition selon l'une quelconque des revendications 1 à 3, **caractérisée en ce que** le polyol est un composé de type alkyle, linéaire ramifié ou cyclique, saturé ou insaturé portant au moins deux fonctions -OH sur la chaine alkyle, ainsi que les polymères (polyéthers) de ces composés alkyles polyhydroxylés.

4. Composition selon la revendication 3, **caractérisée en ce que** le polyol est un composé alkyle ayant de 2 à 12 atomes de carbone, préférentiellement de 2 à 8 atomes de carbone encore plus préférentiellement 2 ou 3 atomes de carbone.

5. Composition selon l'une quelconque des revendications 1 à 4, **caractérisée en ce que** le polyol est choisi parmi l'éthylène glycol [(HOCH₂-CH₂OH)], le diéthylène glycol [(HOCH₂-CH₂-O-CH₂-CH₂OH)], le triéthylène glycol [(HOCH₂-CH₂-O-CH₂-CH₂OCH₂-CH₂OH], le propylène glycol [(propane-1,2-diol : HOCH₂-CHOH-CH₃)], le triméthylène glycol [(propane-1,3-diol : HOCH₂-CH₂-CH₂OH)]; le propyléne glycol, les polymères et les copolymères du glycérol, de l'éthylène glycol et du propylène glycol, comme par exemple le dipropylène glycol et l'hexaglycérol. l'hexylène glycol, le pentylène glycol, le butyldiglycol, le 1,2,3trihydroxyhexane, le butylène glycol [(butane-1,3-diol], le n-butylène glycol [(butane-1,4-diol], le 2,3-butylène glycol [ou secbutylène glycol (butane-2,3 diol)], encore parmi les Triols, par exemple le Glycérol ; les Tétraols comme par exemple l'Érythritol, le Thréitol, ; les Pentols (pentanols) comme par exemple le Xylitol, l'Arabitol (lyxitol), le Ribitol (adonitol) ; les Hexols comme par exemple le Sorbitol (Gulitol), le Dulcitol (Galactitol), le Mannitol, le Fucitol, l'Iditol ; les Heptols comme par exemple le Volemitol ; ou encore l'Isomalt, en C₁₂, le Maltitol, en C₁₂, l'Isomaltitol en C₁₂ le Lactitol (lactositol), en C₁₂, le Maltotriitol, en C₁₈, le Maltotétraitol, en C₂₄ ; le Polyglycitol, Préférentiellement selon l'invention, le polyol peut être choisi parmi triols et hexols, encore plus préférentiellement parmi Glycérol Sorbitol et mannitol.

6. Composition selon l'une quelconque des revendications 1 à 5, **caractérisée en ce que** l'acide hyaluronique est présent dans la composition en une quantité comprise entre 0,01 et 20% du poids total de la composition, préférentiellement entre 0,03% et 10% du poids total de la composition, très préférentiellement entre 0,05% et 1 du poids total de la composition.

7. Composition selon l'une quelconque des revendications 1 à 6, **caractérisée en ce que** le polyol est présent dans la composition en une quantité comprise entre le polyol pourra être présent dans la composition en une quantité comprise entre 0,05 et 90% du poids total de la composition, préférentiellement entre 0,5 et 80% du poids total de la composition, très préférentiellement entre 1,0 et 75% du poids total de la composition.

8. Composition selon l'une quelconque des revendications 1 à 7, **caractérisée en ce que** la carboxyméthylcellulose est présente dans la composition en une quantité comprise entre 0,1% et 72% du poids total de la composition, préférentiellement entre 0,5% et 50% du poids total de la composition, très préférentiellement entre 1 et 5% du poids total de la composition.

9. Composition selon l'une quelconque des revendications 1 à 8, **caractérisée en ce que** le rapport entre l'acide hyaluronique et le polyol est dans la composition compris entre 0,0001 et 400 préférentiellement entre 0,0003 et 2 très préférentiellement entre 0,0006 et 1

10. Composition selon l'une quelconque des revendications 1 à 9, **caractérisée en ce que** le rapport entre l'acide hyaluronique et la carboxyméthylcellulose est dans la composition entre 0,0001 et 200 préférentiellement entre 0,0006 et 20 et très préférentiellement entre 0,01 et 1.

11. Composition selon l'une quelconque des revendications 1 à 10, **caractérisée en ce que** le rapport entre le polyol et la carboxyméthylcellulose est dans la composition compris entre 0,0007 et 900, préférentiellement entre 0,0001 et 160, très préférentiellement entre 0,2 et 75.

12. Utilisation d'au moins un polyol et/ou de carboxyméthylcellulose pour ralentir, limiter, voire annihiler la dégradation de l'acide hyaluronique, dans une composition, particulièrement une composition cosmétique ou pharmaceutique.

13. Utilisation selon la revendication 12, pour ralentir, limiter, voire annihiler la dégradation de l'acide hyaluronique induite par les radiations ionisantes.

14. Utilisation selon la revendication 12, pour ralentir, limiter, voire annihiler la dégradation de l'acide hyaluronique induite par le stress oxydatif.
